# EUROPEAN PATENT SPECIFICATION

(11) **EP 3 697 542 B1**
(45) Date of publication and mention of the grant of the patent: **16.10.2024**
(21) Application number: 18795288.2
(22) Date of filing: 19.10.2018
(51) Int. Cl.: B05B 16/60, B05B 14/43, G01N 33/00

(54) **CONTROL SYSTEM FOR SPRAYING BOOTH**
STEUERUNGSSYSTEM FÜR EINE SPRÜHKABINE
SYSTÈME DE COMMANDE POUR CABINE DE PULVÉRISATION

(30) Priority: 19.10.2017 DK PA201770796
(43) Date of publication of application: 26.08.2020
(73) Proprietor: Carheal X FZCO, DSO-IFZA Dubai (AE)
(72) Inventor: CHRISTENSEN, Henrik Bro, 9530 Støvring (DK)
(74) Representative: TRBL Intellectual Property
(86) International application number: PCT/DK2018/050263
(87) International publication number: WO 2019/076418

(56) References cited:
- WO-A1-2017/152920
- US-A- 4 926 746
- US-A- 5 832 411
- US-A- 6 035 551

## Description

### Field of the Invention

The present invention relates to a control system and a method for one or more spraying booths, which control system comprises at least one VOC detector connected to the control system, which control system performs control of a ventilation system, which ventilation system comprises one or more air circulation means, which ventilation system comprises a filter system, which control system performs mostly continuous storing of at least data representing VOC level and filter status in a cloud based database.

### Background of the Invention

WO 2017/152920 A1 discloses a system for a spraying booth, including a spraying booth and at least one filter with at least one VOC detector, the system further includes a central database where at least data of the VOC detector are stored, and wherein the spraying booth further includes a communication unit with a data connection, whereby the database is cloud based, that the database is further controlled by a controller, that the controller control the spraying booth, where the controller in addition to enabling measuring and documenting of the VOC level in the spraying booth enables controlling all frequency-controlled motors, controlling/measuring/data recording of the spraying booth's LED lighting, motors, emergency stop that causes all movable parts to stop, air renewal in the spraying booth, filtering systems, ventilation systems, controlling/measuring/data recording of other consumption units, hereby controlling/measuring/data recording other data from the spraying booth such as e.g. pressure, temperature, spraying times and drying times.

WO 2014/202084 discloses a system using ultrasound transducers for measuring airflow in a ventilation system.

### Object of the Invention

It is the object of the pending patent application to protect people working in or around a spraying booth.

It is a further object of the pending patent application to reduce pollution in operating a spray booth.

### Description of the Invention

This object can be fulfilled if operation of a spraying booth is performed where during preparation the control system is monitoring the VOC level and the filter status and both have to be accepted by the control system before next step of operation, where during spraying the spraying period is monitored, and by a max spraying period the control system stops spraying, and during spraying the VOC level is monitored, and by max VOC level the control system stops spraying and the control system orders evacuation of the spraying booth, where during ventilation the VOC level is monitored and the VOC level has to be reduced to a VOC safety level before next step of operation, where during heating and drying the VOC level is monitored and ventilation continues, where during end ventilation the VOC level is monitored and after end of the ventilation the system is in a standby situation with a reduced power consumption.

Hereby is achieved that the spraying booth can be operated without polluting the outside surroundings. At the same time operation of the spraying booth for the personnel will be performed in a very safe way because the VOC level can be kept at a relative low level in the spraying booth during operation of any personal. This can be very effective when painting cars where only small sections of the car have to be painted. Hereby it is further effective that the car can be placed in the spraying booth and all preparation automatically takes place until the spraying process has been performed manually. Because there is a control of the maximum spraying period and also the VOC level during spraying, it is possible for the system to stop spraying for example after five minutes and also stop spraying if the VOC level is increasing over a defined level.

In a preferred embodiment for the invention the control system can during spraying shut down at least some of the light sources and as many as possible of other power consumers. Hereby it can be achieved that the power consumption in the spraying booth is reduced but the primary reason for switching off many other consumers is for safety reasons. Heating elements are not to be used because there is a risk of sparks which could dangerous because of the risk of explosion.

Moreover a shutdown of the light sources is a security seeing than an operator is forces to leave the spraying both. Before shutting down the light source a flashing of the light is established as a warning that the light sources will be totally shut down within a short time.

Further there is no reason for more light than the light that is necessary for spraying. Therefore, most of the light can be switched off because of the same reason.

The only power that is to be used during spraying is the power supply to generate pressured air for spraying and for ventilation. Because ventilation is performed by switch mode control of the electro motors, these motors can be formed as asynchronous electro motors where the possibility of generating sparks is totally avoided simply because there is no electric connection between the stator and the rotor.

In a preferred embodiment for the invention the control system comprises at least a first pressure sensor placed at the inlet of the filter system, which control system comprises at least a second pressure sensor placed at the outlet of the filter system, which system calculates the pressure difference between the first and the second pressure sensor for calculating the filter status. Hereby it is possible for the control system to have almost total control of the status of the filter system. The pressure difference will always be a good indication of the effectiveness of any filtration and indicate when the next filter exchange has to be performed. In that way the system is able to predict when the next filter exchange has to be performed and in some situations it will be possible for the control system simply to deny start of the process until the filter is exchanged. It is possible by e.g. active carbon filtration to reduce the level of VOC but other filtration method seems also to be possible. A filter using a combination of adding ozone and UV-light should be able to reduce the content of VOC nearly totally in that the chemical structure of the VOC is changed into environmental friendly components.

In a further preferred embodiment for the invention the spraying device can be connected to an air supply through a supply line, which supply line comprises at least one magnet valve for on/off regulation, which magnetic valve is controlled by the control system. Hereby is achieved that traditional spraying equipment using pressured air in order to vaporise the paint during spraying is controlled by the control system because the magnetic valve has to be open before any pressure is supplied to the spraying unit. Thereby the control system has full control of the spraying process. It is possible in that way that the VOC level in the spraying booth has to be at a low level before spraying is starting simply because the magnetic valve is closed until the correct VOC level is achieved. Further, by increasing the VOC level to a safety level where risk for the operating people exists, the magnetic valve will simply close and stop the spraying process.

In a further preferred embodiment for the invention the control system can control an inlet fan by frequency modulation of the power supply to a fan motor, where a VOC sensor is measuring the VOC level in the inlet air, which VOC sensor is connected to the control system. Hereby can be achieved that the unit that performs the frequency modulation also is able to monitor the power supply of the fan motor and in that way communicate back to the control system if there is any deviation from normal in the power consumption of the fan motor. In the same way it is possible of course to feedback the frequency that is driving the motor and in that way giving the control system information relating to the speed of the fan. In the same way the VOC level can be measured.

In a further preferred embodiment for the invention the control system controls one or more outlet fans by frequency modulation of the power supply to a fan motor, where a VOC sensor is measuring the VOC level in the outlet air, which VOC sensor is connected to the control system. As previously explained/disclosed it is possible to measure the speed of the motor from the control system as well as the power consumption of the motor. Therefore, the operation of the outlet fans is possible. The outlet fan is just as important as control of the inlet fan because by controlling both ventilation systems it is possible also to control the actual pressure in the spraying booth. Hereby can be avoided that VOC gasses are leaving the spraying booth through walls or roof or through smaller openings. By keeping the internal pressure in the spaying booth lower than the outside pressure.

In a further preferred embodiment for the invention airflow generated by the inlet fan and the outlet fan can be increased during the spraying period and continues after end of the spraying activity during a flash off period until a decreasing VOC level is detected.

Hereby it can be achieved that by increasing the speed of the ventilation system, the VOC level is reduced during the spraying period and also just after the spraying, because there is a flash off in a few minutes after end of spraying. This is very important in the flash off period that as many of the VOC gasses is sent to the filters as soon as possible. After the flash off of the volatile gasses and the VOC level is reduced, it is possible to reduce the speed of the ventilation. Further it is possible to let the VOC level more or less control the level of ventilation.

In a further preferred embodiment for the invention the filter system comprises at least one activated carbon filter. Hereby is achieved that most of the VOC gasses will be collected in the activated carbon filter. Further, cleaning of the air that is polluted by VOC gasses could be performed by a combination of ultra violet light and adding ozone to the air passing the filter.

In a further preferred embodiment for the invention can the control system comprise an airflow measuring device for measurement of airflow at least through the activated carbon filter. Hereby can be achieved that the status of the activated carbon filter is under direct control by measuring the actual airflow through the activated carbon filter.

In a further preferred embodiment for the invention can the airflow be measured by a thermic flow sensor. Hereby can be achieved that the airflow will reduce the temperature of a heated wire, where the resistance of the wire is depending of the temperature and the temperature is changing the resistance and the wire. The resistance can be transmitted to the system for indicating the airflow

In a further preferred embodiment for the invention can the airflow measuring be performed by an ultrasound airflow meter comprising one or more ultrasound transducers for generating an ultrasound signal in the airflow. Hereby can the airflow through the activated carbon filter be measured and the actual airflow can be transmitted to the system. A system for measuring airflow in an air duct is published in WO 2014/202084.

In a further preferred embodiment for the invention the control system controls a one-touch cabin mode selector. Hereby can be achieved that in the spraying booth is only one indication because the one-touch cabin mode selector can by change of colour indicate different situations. Green means ready to spray, red could mean time to evacuate and end spraying and in that way the one-touch cabin mode selector is simply the one and only technical installation that is needed inside the spraying booth.

In a further preferred embodiment for the invention the control system controls a UVA/IR heating device. After ending of a spraying process it is necessary that a drying process is performed. Different kind of paint needs different kind of heat treatment. Therefore, it is possible to use as well infrared heating as ultra violet light for heating. In many situations a combination of the two different frequencies of radiation is performed. By local heating of a local painted area it is possible that the drying of the new painted area can be ended in a relative short period.

In a further preferred embodiment for the invention the control system can be connected to at least one sanding equipment. Hereby it is possible to monitor the sanding period that has been used and the control system is able to compare the period used for the sanding with sanding periods stored at the server in the cloud based database.

In a further preferred embodiment for the invention the control system can control opening and closing of a door or gate for access to the spraying booth. Hereby can be achieved that the doors only can be opened in situations where the system accepts door opening but also in emergency situations the control system will be able to open the doors or the gates in order to have a fast ventilation of the inside of a spraying booth.

In a further preferred embodiment for the invention the control system controls one or more light drivers, which light drivers are connected to LED light sources. Hereby is achieved that the control system also controls all the lights that are used inside the spraying booth. The use of LED lighting is positive in that way that light or different frequencies can be achieved. Further is the power consumption of the LEDs reduced.

In a further preferred embodiment for the invention the spraying booth can be a mobile spraying booth for treating cars. Hereby is achieved that the spraying booth maybe can be made as a tent that easily can be moved from one position to another. Hereby it is possible for a short period to operate with a spraying booth e.g. at a large parking area where a number of cars have to be painted for local areas.

In a further preferred embodiment for the invention the treatment includes the following sequence of operation in the said succession: grind, primer, spray, hardening. Hereby is achieved that all the different operations are controlled by the control system and all activities are stored in the cloud based database. Hereby can further be achieved that there can be performed a documentation for each activity.

In a further preferred embodiment for the invention the control system collects data for documentation and administration of the treatment. Hereby it is possible achieve documentation for all treatments that are performed in each of the spraying booths that are connected to the system. In that way it will be possible to guarantee the treatment for all customers. Further, it will be possible to perform statistical analyses of how each single spraying booth is operating and who of the persons operating in the spraying booth are performing.

In a further preferred embodiment for the invention the control system controls at least the consumption of paint, lacquer and other consumables. Hereby is achieved that also the data that refer to the consumption of materials are stored in the same cloud based database.

The pending patent application further concerns a method for control of a spraying booth by monitoring the VOC level and the filter status during preparation, where the control system transmits the VOC level and the filter status to the control system and waits to be accepted by the control system before next step of operation, where the control system monitors the spraying period, where the control system stops the spraying after a defined max spraying period, where the control system monitors during spraying the VOC level, where the control system is stopping the spraying by max VOC level and the control system orders evacuation of the spraying booth, where the control system monitors the VOC level during ventilation and the VOC level has to be reduced to a VOC safety level before next step of operation, where the control system monitors the VOC level during heating and drying and ventilation continues, where during end ventilation is the VOC level monitored.

Hereby can be achieved a very safe way of operating a spraying booth. In the same way the environmental pollution is reduced into the active carbon filters which have to be removed and afterwards most likely handled as normal waste.

### Description of the Drawing

- Fig. 1: shows a sectional view of a spaying booth.
- Fig. 2: shows a control system connected to a spraying booth.
- Fig. 3: shows a possible embodiment for a filter system.
- Fig. 4: shows a circle indicating different operations.
- Fig. 5: shows a top view of a spraying booth.
- Fig. 6: shows the curvature of the VOC level during a possible spraying sequence.
- Fig. 7: indicates a curve of the VOC level in the outlet.
- Fig. 8: shows a sectional view of a flow duct comprising thermal flowmeter.
- Fig. 9: shows a sectional view of a flow duct comprising an ultra sound flowmeter.

### Detailed Description of the Invention

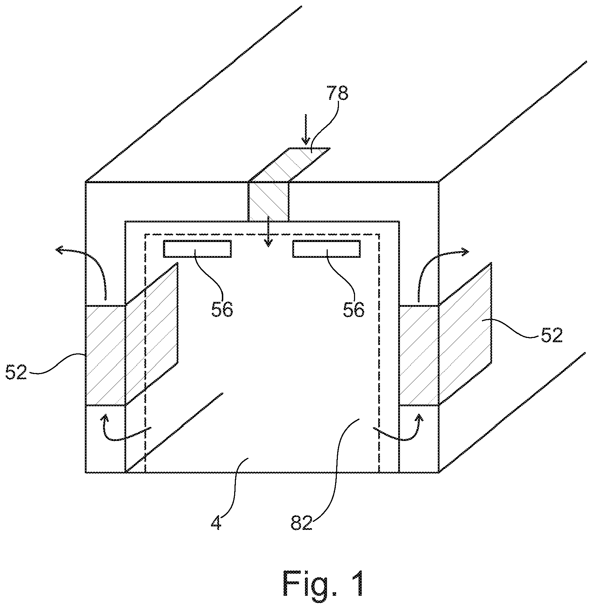

Fig. 1 shows a sectional view of a possible spraying booth 4 where the spraying booth is cut through and you look into a section of the spraying booth 4 where for example doors at both ends are not to be seen. In the walls of the spraying booth 4 activated carbon filters 52 are indicated; here indicated inside the walls. Further is indicated UVA/IR heating devices 56 and further an inlet filter 78 . Inside the spraying booth 4 an explosive zone 82 is indicated.

During operation an item, e.g. a car will be placed inside the spraying booth 4 and during all operation doors would be closed and there will be an isolated climate inside the spraying booth.

Outlet fans and also inlet fans are adjusting the climate inside the spraying booth so that the pressure inside the spraying booth is lower than the pressure outside. This way no VOC gasses will leak from the spraying booth.

During the spraying operation an explosive zone will occur inside the spraying booth simply because the concentration of VOCs together with dust from old paint that could occur inside the spraying booth will form a rather explosive atmosphere.

In order to avoid any kind of explosions the UVA/IR heating device 56 are for example shut off by the system 2 during the spraying process. Further all other electric consumers are more or less shut off during the spraying process. The only technical installations which are in full operation are the VOC sensors and the ventilation means. In this way it is possible to keep the VOC level inside the spraying booth relative low. Because the VOC gasses are sent through the activated carbon filters 52 nearly all VOC particles are kept inside the filters.

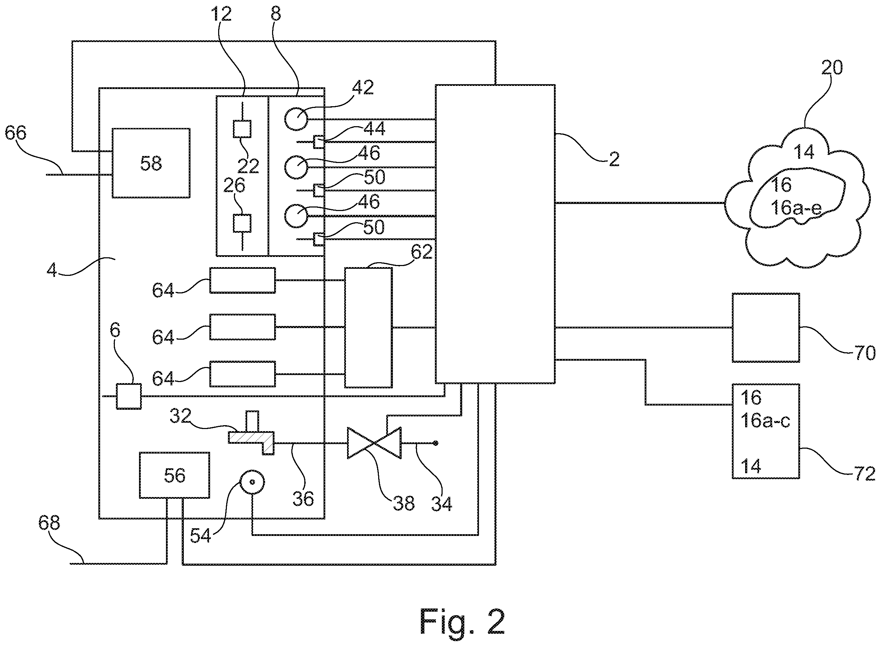

Fig. 2 shows a control system 2 connected to a spraying booth 4. The spraying booth 4 comprises VOC detectors, where VOC means Volatile Organic Compounds which can be detected. Further is indicated a ventilation system 8, which is operating in relation to a filter system 12. Further, fig. 1 shows a cloud based database 20 and a spraying device 32, which spraying device 32 is connected to a supply line 36, which leads to a magnetic valve 38, which is controlled by the control system 2. Further is the valve 38 connected to an air supply 34. Further is indicated an inlet fan motor 42 and a VOC sensor 44. Both are connected to the control system 2. The figure also shows outlet fans 46, which are operating together with VOC sensors 50. All these components are also directly connected to the control system 2. Further is indicated a one-touch cabin mode selector 54 and an UVA/IR heating device 56 is also indicated inside the spraying booth 4 and further sanding equipment 58. The control system 2 is connected to a light controller 62, which light controller 62 is connected to a plurality of LED light sources 64. Further is indicated a power connection 66, which is connected to the sanding equipment 58. Further is showed a power connection 68, which is connected to the UVA/IR heating device 56. Further indicates fig. 1 a customer online dashboard 70 and a support centre 72.

In operation can e.g. a car be placed in a spraying booth 4 because a system of doors can be opened and the car can be placed in the spraying booth 4. After the door is closed the spraying booth is an isolated climatic zone, where the VOC level is monitored by several VOC sensors. VOC sensors are mentioned as 6, 16, 44, 50 and in real life there will probably be even more sensors. The VOC level is monitored by the different sensors, which are all connected to the control system 2. All data that are connected in the control system 2 is further communicated to the cloud based database 20 and the customer online dashboard 70 and to the support centre 72. In operation it is preferred to keep the pressure in the spraying booth below the outside pressure and in that way avoid that any VOC gasses are leaking to the outside. During a spraying operation where spraying equipment 32 is in use, as well the VOC level is measured but also the spraying period. If the VOC level is over a limit or if the spraying period is over a limit, the magnetic valve 38 will be closed by a signal coming from the control system 2. The filter system 12 together with the ventilation system 8 will automatically reduce the VOC level after spraying has stopped, but in a very critical situation where e.g. the VOC level is increasing rapidly, evacuation could be necessary. The control system 2 further controls the inlet fan motor 42, where a VOC sensor 44 is measuring the inlet VOC level. Further is indicated a motor 46 for outlet air. Pressure sensors 22 and 26 are measuring the inlet pressure and the outlet pressure. Further pressure indicators are used in order to measure the pressure difference across every filter that is in use. In that way it is possible to predict the life time of the filters. Further, the control system 2 will perform control via a light driver 62, which is controlling a number of LED sources 64. Hereby it is possible for the control system 2 also to control the light in the spraying booth 4. Light control can be rather important because if painting is performed at different colours it could be rather important to change the spectrum of the light in the spraying booth.

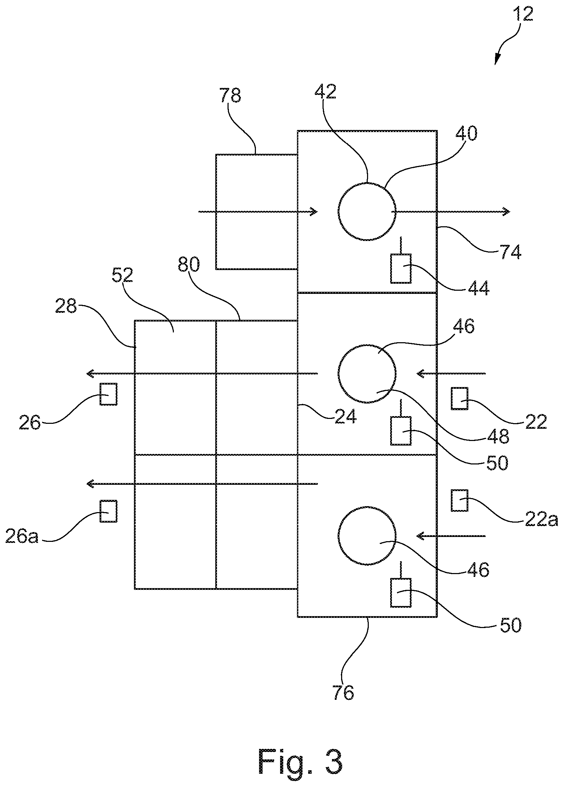

Fig. 3 shows a possible embodiment for a filter system 12. Inlet ventilation means 74 indicates an inlet fan motor 42 which controls the inlet air. The VOC level of the inlet air is indicated by a sensor 44. Further is indicated outlet ventilation means 76 comprising outlet fans 46 driven by motors 48, where the VOC level is indicated by the VOC sensor 50. Further is indicated a pressure sensor 22 placed in the spraying booth 4 and a pressure sensor 26, which is placed outside the spraying booth 4. Hereby the pressure difference can be indicated. Further at fig. 2 is indicated an activated carbon filter 52 and an inlet filter 78 and an outlet pre filter 80.

In operation the inlet air is being filtered and the volume of air is controlled by the fan motor 52 e.g. by frequency modulation. In that way it is possible to perform speed regulation of the ventilation of the inlet air. Further, the outlet air is under control by ventilation 46 driven by a motor 48. This motor is also controlled by frequency modulation and therefore, the speed of the outlet fan can be adjusted from the system 2. The outlet air is at first pre filtered through a pre filter 80 before the outlet air is sent through the activated carbon filter 52. The outlet air 28 is hereafter so clean that the VOC level hardly can be indicated.

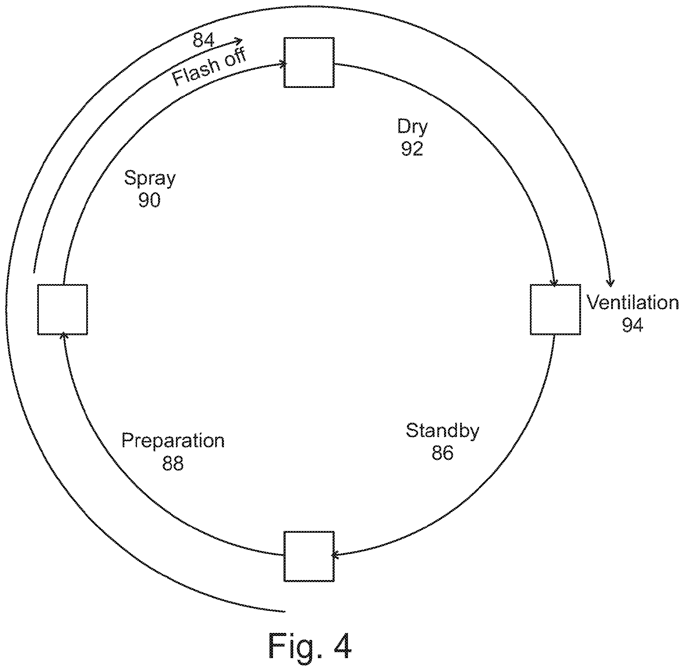

Fig. 4 indicates a circle of different operations which indicates that the system 2 is always operating in a circle as disclosed. Starting by a standby 86 there will be a check indicated by a square until next step which could be a preparation where a sanding operation or other kind of preparation before painting has to be performed. This preparation has to be ended and it has to be checked before going into the next step of operation which is the spraying process 90. It is to be indicated that an outside indication arrow 84 is a flash off indicating that during the spraying process a flash off takes place of VOCs directly from the spraying process or from the surface that is being treated.

There will be a high degree of evaporation of VOCs which has to be removed by the ventilation 94 which is in operation also during the preparation. By end of the spraying process there will be a short waiting process because the flash off period must end before the next step. Therefore, the VOC level has to be accepted before going into the drying process 92.

During the drying process 92 ventilation is still operational. The ventilation is adjustable so that during the flash off period there will be a much higher degree of ventilation as for example during the drying, but even during this process some kind of evaporation of VOCs will still take place from the painted surface. After the drying process the ventilation can be stopped or be substantially reduced. The system can end where it starts in the standby 86 situation. This standby process will reduce the power consumption of the system and the standby period can be very short if the next car has to be treated or a new repair has to be performed maybe at the other side of a car, then the standby operation could end in a few minutes or maybe it could wait until next day.

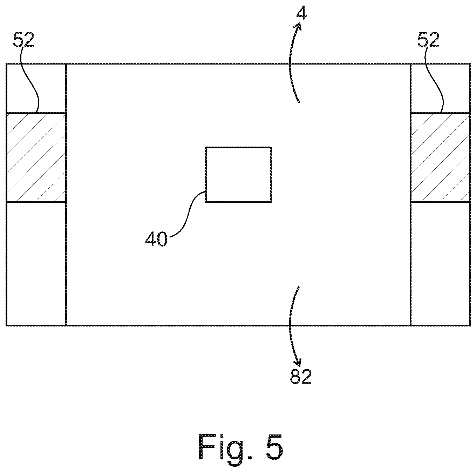

Fig. 5 shows a top view of a spraying booth 4. This indicates that doors are closed and there a closed volume is formed inside the spraying booth 4. Activated carbon filters 52 are indicated in the walls and at the top of the spraying booth 4 is indicated an inlet fan 40 which sends fresh air into the spraying booth 4.

During operation - when doors are closed and spraying is started - there is formed an explosive zone 82 inside the spraying booth 4. This explosive zone exists as long as there is a relative high VOC level in the spraying booth 4. Therefore, during spraying many of the electric consumers operating in the spraying booth are shut off by the system 2.

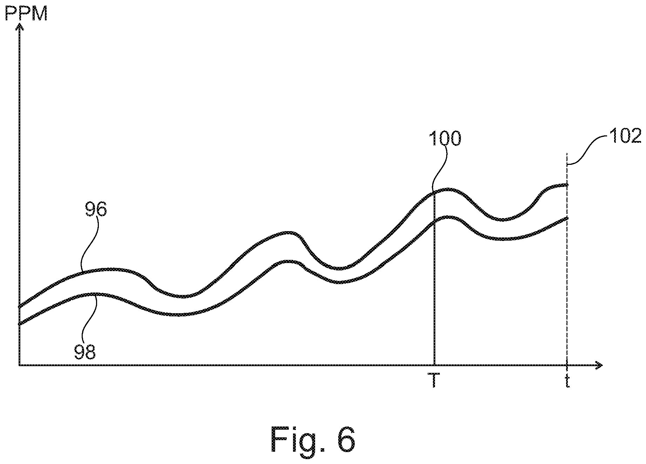

Fig. 6 shows the curvature of the VOC level during a possible spraying sequence. The VOC level in the outlet 96 is indicated by the upper curve and the VOC level in the inlet is indicated by a lower curve 98. These two curves are more or less parallel at very low levels of VOC. A maximum VOC level 100 is indicated where the system probably at first sends a warning to the personnel in the spraying booth 4 and if the VOC level is still increasing, the system will perform an automatic shutdown of the spraying. Further is indicated the maximum spraying period 102.

The spraying booth is typically used for repair of cars. Therefore, the spraying period will typical have a maximum of 10 minutes and in many situations the spraying process is finished in shorter time.

For safety reasons there is a shutdown if the VOC level is over the safety level and also an automatic stop if the maximum spraying period is reached.

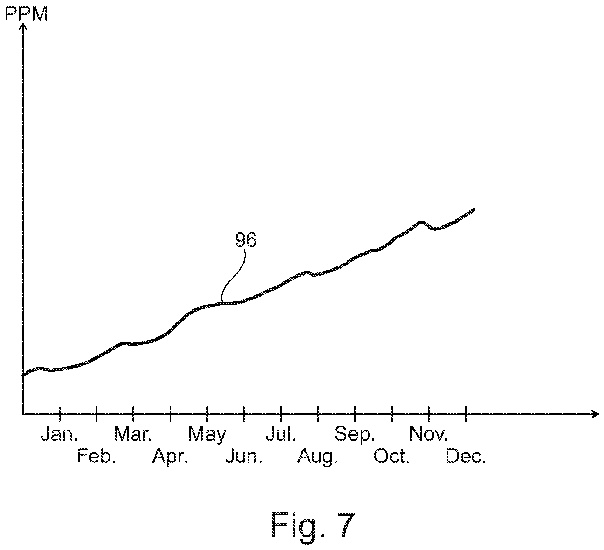

Fig. 7 indicates a curve of the VOC level in the outlet 96 which curve is increasing during a very long period. This way it is possible to indicate the maximum service time of the activated carbon filters 52. Further the system is also performing measurement of the pressure difference over the activated carbon filters 52. This information about increasing pressure difference together with increasing VOC level is also indicating that it is time to exchange the activated carbon filters 52.

Because there is a check of the activity of the activated carbon filters 52 it is possible to predict the period for exchange of the activated carbon filters.

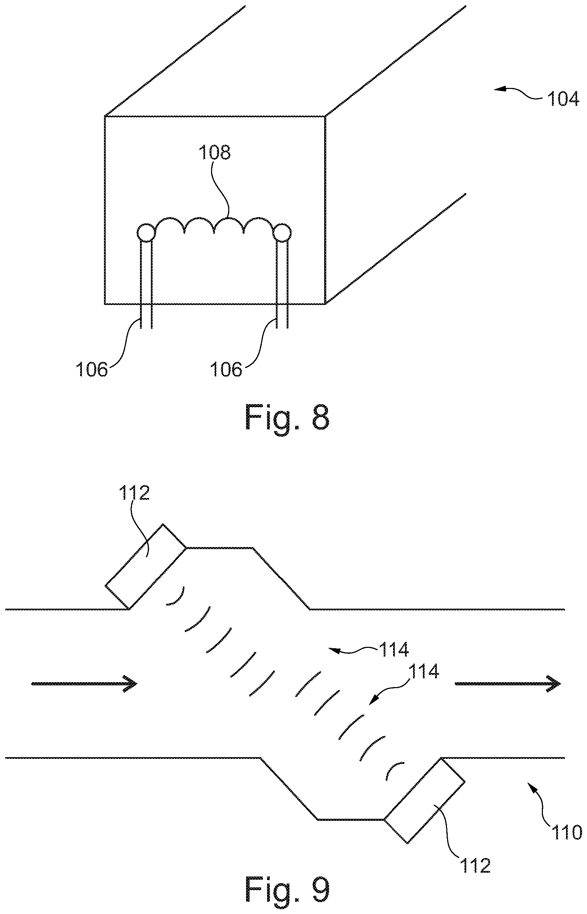

Fig. 8 discloses a sectional view of a flow duct 104. In the flow duct is indicated a terminal 106 carrying a heated wire 108.

The temperature of the wire 108 is indicating airflow. The temperature of the wire can be measured by measuring the resistance in the wire. Hereby can the system perform measuring of the flow through the activated carbon filter.

Fig. 9 discloses a sectional view of a flow duct 110 comprising ultrasound transducers 112 transmitting ultrasound 114 between the ultrasound transducers.

Hereby is achieved that the airflow is measured very effectively in the flow duct 110. More ultrasound transducers can be used for measuring more of the volume in the flow duct for achieving a better result.

### List of reference numbers

Control system (2)
spraying booths (4)
VOC detector (6), VOC (Volatile Organic Compounds)
ventilation system (8)
air circulation means (10)
filter system (12)
data (14) representing VOC level (16) (16a) (16b) (16d) (16e)
filter status (18)
cloud based database (20)
first pressure sensor (22)
inlet (24) of the filter system (12)
second pressure sensor (26)
outlet (28) of the filter system (12)
pressure difference (30)
spraying device (32)
air supply (34)
supply line (36)
magnet valve (38)
inlet fan (40)
fan motor (42)
VOC sensor (44)
outlet fans (46)
fan motor (48)
VOC sensor (50)
activated carbon filter (52)
one-touch cabin mode selector (54)
UVA/IR heating device (56)
sanding equipment (58)
door (60) gate (60a)
light drivers (62)
LED light sources (64)
power connection (66)
power connection (68)
customer online dashboard (70)
support centre (72)
inlet ventilation means (74)
outlet ventilation means (76)
inlet filter (78)
outlet pre filter (80)
explosive zone (82)
flash off (84)
standby (86)
preparation (88)
spray (90)
dry (92)
ventilation (94)
VOC level in the outlet (96)
VOC level in the inlet (98)
VOC max level (100)
max spray period (102)
flow duct (104)
electric terminal (106)
heated wire (108)
flow duct (110)
ultrasound transducer (112)
ultrasound (114)

## Claims

1. A control system (2) for one or more spraying booths (4), which control system (2) comprises at least one VOC detector (6) connected to the control system (2), which control system (2) comprises a ventilation system (8) and is configured to perform control of the ventilation system (8), which ventilation system comprises one or more air circulation means (10), which ventilation system (8) comprises a filter system (12), which control system (2) performs mostly continuous storing of at least data (14) representing VOC level (16) and filter status (18) in a cloud based database (20), **characterized in that**
- the control system (2) is configured to monitor the VOC level (16a),
- the control system (2) is configured to check and accept the VOC level (16a) and the filter status (18) before allowing start of spraying in the spraying booth (4),
- the control system is configured to monitor the spraying period (90) during spraying and is configured to stop the spraying when a max spraying period is received
- the control system is configured to monitor the VOC level (16a) during spraying (90) and is configured to stop the spraying and to order evacuation of the spraying booth when a max VOC level (16b) is received,
- the control system is configured to monitor the VOC level (16a) during ventilation and is configured to check that the VOC level (16a) is reduced to a VOC safety level (16c) before start of drying (92),
- the control system is configured to monitor the VOC level (16a) during heating and drying (92) and is configured to let ventilation (94) continue,
- the control system is configured to monitor the VOC level (16a) during the end of ventilation (94), and
- the control system is configured to enter in a standby (86) situation with a reduced power consumption after the end of the ventilation (94).

2. Control system according to claim 1, **characterized in that** the control system (2) during spraying (90) shuts down at least some of the light sources (56,64) and as many as possible of other power consumers.

3. Control system according to claim 1 or 2, **characterized in that** the control system (2) comprises at least a first pressure sensor (22) placed at the inlet (24) of the filter system (12), which control system (2) comprises at least a second pressure sensor (26) placed at the outlet (28) of the filter system (12), which system calculates the pressure difference (30) between the first (22) and the second pressure sensors (26) for calculating the filter status (18).

4. Control system according to one of claims 1-3, **characterized in that** it comprises a spraying device (32) connected to an air supply (34) through a supply line (36), said supply line (36) comprises at least one magnet valve (38) for on/off regulation, said control system (2) being further configured to control said magnet valve (38) for on/off regulation of said air supply line (36).

5. Control system according to one of the claims 1-4, **characterized in that** the control system (2) is configured to control an inlet fan (40) of the air circulation means (10) by frequency modulation of the power supply to a fan motor (42), where a VOC sensor (44) is measuring the VOC level (16d) in the inlet air, which VOC sensor (44) is connected to the control system (2).

6. Control system according to one of the claims 1-5, **characterized in that** the one or more air circulation means (10) comprises an inlet fan (40) and an outlet fan (46), wherein the inlet fan (40) and the outlet fan (46) are configured to generate an airflow which is increased during the spray period and continues after end of spray activity during a flash off (84) period until a decreasing VOC level (16d) is detected.

7. Control system according to one of the claims 1-6, **characterized in that** the control system (2) is configured to control one or more outlet fans (46) by frequency modulation of the power supply to a fan motor (48), where a VOC sensor (50) is measuring the VOC level (16e) in the outlet air, which VOC sensor (50) is connected to the control system (2).

8. Control system according to one of the claims 1-7, **characterized in that** the filter system (12) comprises at least one activated carbon filter (52).

9. Control system according to one of the claims 1-8, **characterized in that** the control system comprises an airflow measuring device for measurement of airflow at least through the activated carbon filter (52).

10. Control system according to claim 9, **characterized in that** the airflow measuring device is a thermic flow sensor or an ultrasound airflow meter comprising one or more ultrasound transducers for generating an ultrasound signal in the airflow.

11. Control system according to one of the preceding claims, **characterized in that** the control system (2) controls a UVA/IR heating device (56).

12. Control system according to one of the preceding claims, **characterized in that** the control system (2) controls a mechanism for opening and closing of a door (60) or gate (60a) for access to the spraying booth (4).

13. Method for control of a spraying booth (4) with a control system according to any of the preceding claims, **characterized in** at least the following steps:
a. monitor the VOC level (16a) and the filter status (18) during preparation,
b. transmit the VOC level (16a) and the filter status (18) to the control system (2) and wait for acceptance by the control system (2) before start spraying,
c. let the control system (2) monitor the spraying period (20),
d. the control system (2) stops the spraying after a defined max spraying period (20a),
e. the control system monitors during spraying the VOC level (16a),
f. the control system (2) is stopping the spraying by max VOC level (16b) and the control system (2) orders evacuation of the spraying booth (4),
g. the control system monitors the VOC level (16a) during ventilation and the VOC level (16a) has to be reduced to a VOC safety level (16c) before start of heating,
h. the control system monitors the VOC level (16a) during heating and drying and ventilation continues,
i. during end ventilation is the VOC level (16a) monitored,
j. when the VOC level (16a) has reached a safety level the system enters a standby mode of operation.

## Patentansprüche

1. Steuerungssystem (2) für eine oder mehrere Sprühkabinen (4), wobei das Steuerungssystem (2) mindestens einen VOC-Detektor (6) umfasst, der mit dem Steuerungssystem (2) verbunden ist, wobei das Steuerungssystem (2) ein Belüftungssystem (8) umfasst und dazu konfiguriert ist, das Steuern des Belüftungssystems (8) durchzuführen, wobei das Belüftungssystem ein oder mehrere Luftzirkulationsmittel (10) umfasst, wobei das Belüftungssystem (8) ein Filtersystem (12) umfasst, wobei das Steuerungssystem (2) eine größtenteils kontinuierliche Speicherung von mindestens Daten (14), die das VOC-Niveau (16) und den Filterstatus (18) darstellen, in einer Cloud-basierten Datenbank (20) durchführt, **dadurch gekennzeichnet, dass**
- das Steuerungssystem (2) dazu konfiguriert ist, das VOC-Niveau (16a) zu überwachen,
- das Steuerungssystem (2) dazu konfiguriert ist, das VOC-Niveau (16a) und den Filterstatus (18) zu überprüfen und zu akzeptieren, bevor das Starten des Sprühens in der Sprühkabine (4) erlaubt wird,
- das Steuerungssystem dazu konfiguriert ist, die Sprühperiode (90) während des Sprühens zu überwachen und dazu konfiguriert ist, das Sprühen zu stoppen, wenn eine maximale Sprühperiode empfangen wird
- das Steuerungssystem dazu konfiguriert ist, das VOC-Niveau (16a) während des Sprühens (90) zu überwachen, und dazu konfiguriert ist, das Sprühen zu stoppen und das Evakuieren aus der Sprühkabine anzuordnen, wenn ein maximales VOC-Niveau (16b) empfangen wird,
- das Steuerungssystem dazu konfiguriert ist, das VOC-Niveau (16a) während der Belüftung zu überwachen und dazu konfiguriert ist, zu überprüfen, ob das VOC-Niveau (16a) auf ein VOC-Sicherheitsniveau (16c) reduziert wird, bevor das Trocknen (92) startet,
- das Steuerungssystem dazu konfiguriert ist, das VOC-Niveau (16a) während des Erwärmens und Trocknens (92) zu überwachen, und dazu konfiguriert ist, die Belüftung (94) fortzusetzen,
- das Steuerungssystem dazu konfiguriert ist, das VOC-Niveau (16a) während des Endes der Belüftung (94) zu überwachen, und
- das Steuerungssystem dazu konfiguriert ist, nach dem Ende der Belüftung (94) in eine Bereitschaftssituation (86) mit reduziertem Stromverbrauch einzutreten.

2. Steuerungssystem nach Anspruch 1, **dadurch gekennzeichnet, dass** das Steuerungssystem (2) während des Sprühens (90) mindestens einige der Lichtquellen (56, 64) und so viele wie möglich von anderen Stromverbrauchern abschaltet.

3. Steuerungssystem nach Anspruch 1 oder 2, **dadurch gekennzeichnet, dass** das Steuerungssystem (2) mindestens einen ersten Drucksensor (22) umfasst, der am Einlass (24) des Filtersystems (12) platziert ist, wobei das Steuerungssystem (2) mindestens einen zweiten Drucksensor (26) umfasst, der am Auslass (28) des Filtersystems (12) platziert ist, wobei das System die Druckdifferenz (30) zwischen dem ersten (22) und dem zweiten Drucksensor (26) berechnet, um den Filterstatus (18) zu berechnen.

4. Steuerungssystem nach einem der Ansprüche 1-3, **dadurch gekennzeichnet, dass** es eine Sprühvorrichtung (32) umfasst, die über eine Zufuhrleitung (36) mit einer Luftzufuhr (34) verbunden ist, wobei die Zufuhrleitung (36) mindestens ein Magnetventil (38) zur Ein-/Aus-Regulierung umfasst, wobei das Steuerungssystem (2) ferner dazu konfiguriert ist, das Magnetventil (38) zur Ein-/Aus-Regulierung der Luftzufuhrleitung (36) zu steuern.

5. Steuerungssystem nach einem der Ansprüche 1-4, **dadurch gekennzeichnet, dass** das Steuerungssystem (2) dazu konfiguriert ist, ein Einlassgebläse (40) des Luftzirkulationsmittels (10) durch Frequenzmodulation der Stromversorgung zu einem Gebläsemotor (42) zu steuern, wobei ein VOC-Sensor (44) das VOC-Niveau (16d) in der Einlassluft misst, wobei der VOC-Sensor (44) mit dem Steuerungssystem (2) verbunden ist.

6. Steuerungssystem nach einem der Ansprüche 1-5, **dadurch gekennzeichnet, dass** das eine oder die mehreren Luftzirkulationsmittel (10) ein Einlassgebläse (40) und ein Auslassgebläse (46) umfasst, wobei das Einlassgebläse (40) und das Auslassgebläse (46) dazu konfiguriert sind, einen Luftstrom zu generieren, der während der Sprühperiode verstärkt wird und nach dem Ende der Sprühaktivität während einer Ablüftperiode (84) anhält, bis ein abnehmendes VOC-Niveau (16d) detektiert wird.

7. Steuerungssystem nach einem der Ansprüche 1-6, **dadurch gekennzeichnet, dass** das Steuerungssystem (2) dazu konfiguriert ist, ein oder mehrere Auslassgebläse (46) durch Frequenzmodulation der Stromversorgung zu einem Gebläsemotor (48) zu steuern, wobei ein VOC-Sensor (50) das VOC-Niveau (16e) in der Auslassluft misst, wobei der VOC-Sensor (50) mit dem Steuerungssystem (2) verbunden ist.

8. Steuerungssystem nach einem der Ansprüche 1-7, **dadurch gekennzeichnet, dass** das Filtersystem (12) mindestens einen Aktivkohlefilter (52) umfasst.

9. Steuerungssystem nach einem der Ansprüche 1-8, **dadurch gekennzeichnet, dass** das Steuerungssystem eine Luftstrom-Messvorrichtung zum Messen des Luftstroms mindestens durch den Aktivkohlefilter (52) umfasst.

10. Steuerungssystem nach Anspruch 9, **dadurch gekennzeichnet, dass** die Luftstrom-Messvorrichtung ein thermischer Stromsensor oder ein Ultraschall-Luftstrommesser ist, umfassend einen oder mehrere Ultraschallwandler zum Generieren eines Ultraschallsignals im Luftstrom.

11. Steuerungssystem nach einem der vorhergehenden Ansprüche, **dadurch gekennzeichnet, dass** das Steuerungssystem (2) eine UVA/IR-Heizvorrichtung (56) steuert.

12. Steuerungssystem nach einem der vorhergehenden Ansprüche, **dadurch gekennzeichnet, dass** das Steuerungssystem (2) einen Mechanismus zum Öffnen und Schließen einer Tür (60) oder eines Tors (60a) für den Zugang zur Sprühkabine (4) steuert.

13. Verfahren zum Steuern einer Sprühkabine (4) mit einem Steuerungssystem nach einem der vorhergehenden Ansprüche, **gekennzeichnet durch** mindestens die folgenden Schritte:
a. Überwachen des VOC-Niveaus (16a) und des Filterstatus (18) während der Vorbereitung,
b. Übertragen des VOC-Niveaus (16a) und des Filterstatus (18) an das Steuerungssystem (2) und Warten auf das Akzeptieren durch das Steuerungssystem (2), bevor das Sprühen gestartet wird,
c. Ermöglichen, dass das Steuerungssystem (2) die Sprühperiode (20) überwacht,
d. Stoppen des Sprühens durch das Steuerungssystem (2) nach einer definierten maximalen Sprühperiode (20a),
e. Überwachen des VOC-Niveaus (16a) durch das Steuerungssystem während des Sprühens,
f. Stoppen durch das Steuerungssystem (2) des Sprühens durch das maximale VOC-Niveau (16b) und Anordnen durch das Steuerungssystem (2) des Evakuierens der Sprühkabine (4),
g. Überwachen des VOC-Niveaus (16a) durch das Steuerungssystem während der Belüftung und Reduzieren des VOC-Niveaus (16a) auf ein VOC-Sicherheitsniveau (16c), bevor das Erwärmen gestartet wird,
h. Überwachen des VOC-Niveaus (16a) während des Erwärmens und Trocknens durch das Steuerungssystem und Fortsetzen der Belüftung,
i. Überwachen des VOC-Niveaus (16a) während der Endlüftung,
j. Eintreten des Systems in einen Bereitschaftsbetriebsmodus, wenn das VOC-Niveau (16a) ein Sicherheitsniveau erreicht hat.

## Revendications

1. Un système de contrôle (2) pour une ou plusieurs cabines de pulvérisation (4), lequel système de commande (2) comprend au moins un détecteur de COV (6) connecté au système de commande (2), lequel système de commande (2) comprend un système de ventilation (8) et est configuré pour effectuer la commande du système de ventilation (8), lequel système de ventilation comprend un ou plusieurs moyens de circulation d'air (10), lequel système de ventilation (8) comprend un système de filtrage (12), lequel système de commande (2) effectue principalement un stockage continu d'au moins des données (14) représentant le niveau de COV (16) et l'état du filtre (18) dans une base de données basée sur le nuage (20), **caractérisée en ce que**
- le système de commande (2) est configuré pour surveiller le niveau de COV (16a),
- le système de commande (2) est configuré pour vérifier et accepter le niveau de COV (16a) et l'état du filtre (18) avant d'autoriser le début de la pulvérisation dans la cabine de pulvérisation (4),
- le système de commande est configuré pour surveiller la période de pulvérisation (90) pendant la pulvérisation et est configuré pour arrêter la pulvérisation lorsqu'une période de pulvérisation maximale est reçue,
- le système de commande est configuré pour surveiller le niveau de COV (16a) pendant la pulvérisation (90) et est configuré pour arrêter la pulvérisation et ordonner l'évacuation de la cabine de pulvérisation lorsqu'un niveau maximal de COV (16b) est reçu,
- le système de commande est configuré pour surveiller le niveau de COV (16a) pendant la ventilation et est configuré pour vérifier que le niveau de COV (16a) est réduit à un niveau de sécurité de COV (16c) avant le début du séchage (92),
- le système de commande est configuré pour surveiller le niveau de COV (16a) pendant le chauffage et le séchage (92) et est configuré pour laisser la ventilation (94) se poursuivre,
- le système de commande est configuré pour surveiller le niveau de COV (16a) pendant la fin de la ventilation (94), et
- le système de commande est configuré pour entrer dans une situation de veille (86) avec une consommation d'énergie réduite après la fin de la ventilation (94).

2. Système de commande selon la revendication 1, **caractérisé en ce que** le système de commande (2) arrête, pendant la pulvérisation (90), au moins certaines des sources lumineuses (56,64) et le plus grand nombre possible d'autres consommateurs d'énergie.

3. Système de commande selon la revendication 1 ou 2, **caractérisé en ce que** le système de commande (2) comprend au moins un premier capteur de pression (22) placé à l'entrée (24) du système de filtration (12), lequel système de commande (2) comprend au moins un second capteur de pression (26) placé à la sortie (28) du système de filtration (12), lequel système calcule la différence de pression (30) entre le premier (22) et le second capteur de pression (26) pour calculer l'état du filtre (18).

4. Système de commande selon l'une des revendications 1 à 3, **caractérisé en ce qu'**il comprend un dispositif de pulvérisation (32) relié à une alimentation en air (34) par une conduite d'alimentation (36), ladite conduite d'alimentation (36) comprend au moins une vanne magnétique (38) pour la régulation marche/arrêt, ledit système de commande (2) étant en outre configuré pour commander ladite vanne magnétique (38) pour la régulation marche/arrêt de ladite conduite d'alimentation en air (36).

5. Système de commande selon l'une des revendications 1 à 4, **caractérisé en ce que** le système de commande (2) est configuré pour contrôler un ventilateur d'entrée (40) du moyen de circulation d'air (10) par modulation de fréquence de l'alimentation électrique d'un moteur de ventilateur (42), où un capteur de COV (44) mesure le niveau de COV (16d) dans l'air d'entrée, ce capteur de COV (44) étant connecté au système de contrôle (2).

6. Système de commande selon l'une des revendications 1 à 5, **caractérisé par le fait que** le ou les moyens de circulation d'air (10) comprennent un ventilateur d'entrée (40) et un ventilateur de sortie (46), le ventilateur d'entrée (40) et le ventilateur de sortie (46) étant configurés pour générer un flux d'air qui augmente pendant la période de pulvérisation et se poursuit après la fin de l'activité de pulvérisation pendant une période d'extinction (84) jusqu'à ce qu'un niveau décroissant de COV (16d) soit détecté.

7. Système de commande selon l'une des revendications 1 à 6, **caractérisé en ce que** le système de commande (2) est configuré pour contrôler un ou plusieurs ventilateurs de sortie (46) par modulation de fréquence de l'alimentation électrique d'un moteur de ventilateur (48), lorsqu'un capteur de COV (50) mesure le niveau de COV (16e) dans l'air de sortie, ce capteur de COV (50) étant connecté au système de commande (2).

8. Système de commande selon l'une des revendications 1 à 7, **caractérisé en ce que** le système de filtration (12) comprend au moins un filtre à charbon actif (52).

9. Système de commande selon l'une des revendications 1 à 8, **caractérisé en ce que** le système de commande comprend un dispositif de mesure du débit d'air pour mesurer le débit d'air au moins à travers le filtre à charbon actif (52).

10. Système de contrôle selon la revendication 9, **caractérisé en ce que** le dispositif de mesure du débit d'air est un capteur de débit thermique ou un débitmètre d'air à ultrasons comprenant un ou plusieurs transducteurs à ultrasons pour générer un signal ultrasonore dans le débit d'air.

11. Système de commande selon l'une des revendications précédentes, **caractérisé en ce que** le système de commande (2) commande un dispositif de chauffage UVA/IR (56).

12. Système de commande selon l'une des revendications précédentes, **caractérisé en ce que** le système de commande (2) commande un mécanisme d'ouverture et de fermeture d'une porte (60) ou d'un portail (60a) d'accès à la cabine de pulvérisation (4).

13. Procédé de commande d'une cabine de pulvérisation (4) avec un système de commande selon l'une quelconque des revendications précédentes, **caractérisé par** au moins les étapes suivantes :
a. surveiller le niveau de COV (16a) et l'état du filtre (18) pendant la préparation,
b. transmettre le niveau de COV (16a) et l'état du filtre (18) au système de commande (2) et attendre l'acceptation du système de contrôle (2) avant de commencer la pulvérisation,
c. laisser le système de contrôle (2) surveiller la période de pulvérisation (20),
d. le système de commande (2) arrête la pulvérisation après une période de pulvérisation maximale définie (20a),
e. le système de commande surveille le niveau de COV (16a) pendant la pulvérisation,
f. le système de commande (2) arrête la pulvérisation en fonction du niveau maximal de COV (16b) et le système de commande (2) ordonne l'évacuation de la cabine de pulvérisation (4),
g. le système de commande surveille le niveau de COV (16a) pendant la ventilation et le niveau de COV (16a) doit être ramené à un niveau de sécurité de COV (16c) avant le début du chauffage,
h. le système de commande surveille le niveau de COV (16a) pendant le chauffage et le séchage et la ventilation continue,
i. le niveau de COV (16a) est contrôlé pendant la ventilation finale,
j. lorsque le niveau de COV (16a) a atteint un niveau de sécurité, le système passe en mode d'attente.
